Europäisches Patentamt

European Patent Office

Office européen des brevets

(19)

(11) **EP 0 893 128 B1**

(12) **EUROPEAN PATENT SPECIFICATION**

(45) Date of publication and mention
of the grant of the patent:
**19.05.2004   Bulletin 2004/21**

(51) Int Cl.[7]: **A61L 9/01**, A61L 9/03,
A61L 9/12

(21) Application number: **98304787.9**

(22) Date of filing: **17.06.1998**

(54) **Composite space deodorizing filter**

Verbundluftdesodorierungsfilter

Filtre désodorisant composite pour l'air

(84) Designated Contracting States:
**DE FR GB**

(30) Priority: **23.06.1997  JP 16612297**
           **28.08.1997  JP 23223497**

(43) Date of publication of application:
**27.01.1999   Bulletin 1999/04**

(73) Proprietor: **SHARP KABUSHIKI KAISHA**
**Osaka-shi, Osaka-fu 545-0013 (JP)**

(72) Inventors:
• **Watsuji, Toru**
**Matsudo-shi, Chiba (JP)**
• **Miyata, Akio**
**Abiko-shi, Chiba (JP)**
• **Nojima, Hideo**
**Abiko-shi, Chiba (JP)**

(74) Representative: **West, Alan Harry et al**
**R.G.C. Jenkins & Co.**
**26 Caxton Street**
**London SW1H 0RJ (GB)**

(56) References cited:
**WO-A-96/00019          US-A- 4 956 183**

• **PATENT ABSTRACTS OF JAPAN vol. 014, no.**
**068 (C-0686), 8 February 1990 (1990-02-08) & JP**
**01 288318 A (NIPPON CARBIDE IND CO INC), 20**
**November 1989 (1989-11-20)**
• **PATENT ABSTRACTS OF JAPAN vol. 017, no.**
**606 (C-1128), 8 November 1993 (1993-11-08) & JP**
**05 184648 A (MITSUBISHI ELECTRIC CORP), 27**
**July 1993 (1993-07-27)**
• **DATABASE WPI Section Ch, Week 199250**
**Derwent Publications Ltd., London, GB; Class**
**E12, AN 1992-413579 XP002145268 & SU 1 703**
**650 A (IVAN CHEM TECHN INST), 7 January 1992**
**(1992-01-07)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to a deodorizing filter for eliminating odor in air, which is used for electric appliances, mainly including, air conditioners.

Enhanced domestic comfort has been demanded with the increased quality of the environment. In modern air-tight houses, however, odor is liable to stay. The need to eliminate odors of cigarettes, sweat, infants and pets has been increased. To this end, proposals to incorporate filters having a deodorizing or odor eliminating capability in air conditioners including air purifiers, have been made. For such deodorizing purposes activated carbon, photo-catalytic oxidation or ozone oxidation is usually used.

Activated carbon filters have a problem in that the adsorbing capability is temporal so that odor molecules which have been adsorbed in the activated carbon will be removed for release at equilibrium since the filter has a limited adsorbing power although it eliminates odor in the atmosphere by adsorbing odor molecules.

In order to overcome this problem, methods of deodorizing with metallic catalyst oxidation using platinum and nickel while recovering activated carbon by heat treatment have been proposed (for example, Japanese Laid-open Patent Publication No. 63-240923 and No. 3-224619). However, it is constantly necessary to heat the activated carbon to about 300 to 400 c to conduct such metal catalytic oxidation. There is the risk that the activated carbon may be ignited at high temperatures. If the deodorizing filter is used for a consumer's electrical appliances other than cooking apparatus and warming apparatus, the electric power consumption would be high or the apparatus should be provided with heat insulation. Since the heating temperature is high, separating rate would become higher than that of catalyst so that odor can not be sufficiently eliminated. Odor molecules and molecules which are generated by a catalyst will react with each other at elevated temperatures so that harmful components and new odor may be generated.

In the deodorizing method using photo-catalytic oxidation, deodorizing is slow and it is necessary to provide an excitation light source. Use of lamps provides problems of high cost and high electric power consumption.

In the ozone oxidation method, an apparatus for decomposing excess ozone is necessary. It provides problems such as high cost, high electric power consumption, as well as safety due to the fact that ozone is per se harmful to the human body.

As the method of solving the problems of the above-mentioned methods, an oxidation catalyst using a transition metal chelate compound such as iron phthalocyanine complex attracts attention. This method is described in, for example, Hirofusa Shirai, "Kagaku to Kogyo", p.27, Oct. 1990; and Naoki Toshima, "Kogyo Zairyo", p.45, Oct, 1991. A deodorizer utilizing the oxidation and reduction ability of metal phthalocyanine is disclosed in Japanese Laid-open Patent Publication No. 62-97555. Since many odor molecules have mobile hydrogen atoms, deodorization is possible by oxidation by dehydrogenation; dimerization, rendering it water-soluble and making it nonvolatile.

This deodorizing method can be exemplified by the action of an in-vivo enzyme upon odor molecules. Catalase which performs these reactions contains hematoporphyrin, which is bonded to apoprotein. Its ferric iron atoms and histidineimidazole nitrogen atoms of the protein become 5-coordinated. The carboxyphthalocyanine iron complex having a catalytic activity similar to that of catalase, such as the octacarboxyphthalocyanine iron complex will decompose in accordance with a reaction mechanism similar to catalase and has a catalytic activity which is six times as high as hemin. An example is the oxidation of mercaptan represented as follows:

$$2\text{-R-SH} + 2\text{OH}^- \rightarrow 2\text{R-S}^- + 2\text{H}_2\text{O} \tag{1}$$

$$2\text{R-S}^- + 2\text{H}_2\text{O} + \text{O}_2 \rightarrow \text{R-S-S-R} + \text{H}_2\text{O}_2 + 2\text{OH}^- \tag{2}$$

wherein R denotes $CH_3$ or $C_2H_5$.

Chelate anions which are formed by reaction (1) will be coordinated at metal phthalocyanine so that it becomes an active tertiary complex. Chelate anions which are coordinated at this active species are dimerized into disulfide so that deodorization is achieved.

However, the transition metal chelate compound has a problem that the deodorizing speed is lower than activated carbon and its deodorizing effect is remarkably low in an environment such as air conditioner in which the air flows over the deodorizing element.

The present invention now provides a composite deodorizing filter having deodorizing means including an adsorbent for odor molecules and a transition metal chelate for catalysing oxidation of odor molecules and characterized by including also air permeable heating means integrated with and capable of heating the deodorizing means.

In one preferred embodiment, the composite deodorizing filter according to the invention comprises powdery or granular adsorbent bonded to the heating means and the transition metal chelate is carried on the heating means and the adsorbent.

In a second preferred embodiment of the composite deodorizing filter according to the invention, the deodorizing means comprises powdery or granular adsorbent and powdery or granular transition metal chelate and the deodorizing means is contained in a casing mounted on the heating means.

In a third preferred embodiment of the composite deodorizing filter according to the invention, the deodorizing means comprises powdery or granular adsorbent which carries the transition metal chelate and the deodorizing means is contained in a casing mounted on the heating means.

In a fourth preferred embodiment of the composite deodorizing filter according to the invention, the deodorizing means comprises an air permeable adsorbent-containing body and an air permeable transition metal chelate-containing body and the heating means, the adsorbent-containing body and the transition metal chelate-containing body are arranged in sequence.

In a fifth preferred embodiment of the composite deodorizing filter according to the invention, the deodorizing means comprises an air permeable adsorbent-containing body and an air permeable transition metal chelate-containing body and a first transition metal chelate-containing body, a first adsorbent-containing body, the heating means, a second adsorbent-containing body and a second transition metal chelate-containing body are arranged in sequence.

In a sixth preferred embodiment of the composite deodorizing filter according to the invention, an air permeable adsorbent-containing body is bonded to the heating means and an air permeable transition metal chelate-containing body is wrapped around the adsorbent-containing body and the heating means.

In another preferred embodiment of the composite deodorizing filter according to the invention, the heating means is sandwiched between two air permeable adsorbent-containing bodies and an air permeable transition metal chelate-containing body is wrapped around the sandwich.

In a further preferred embodiment of the composite deodorizing filter according to the invention, an air permeable adsorbent-containing body is wrapped around the heating means and an air permeable transition metal chelate-containing body is wrapped around the adsorbent-containing body.

The deodorizing filter of the invention may be produced, for example, by dissolving a polycarboxylic acid metal-phthalocyanine in alkaline aqueous solution, deoxygenating the solution (preferably by bubbling through a gas, suitably an inert gas such as nitrogen or argon), dipping an air-permeable body such as a filter of, for example, unwoven fabric, paper honeycomb, ceramic fiber honeycomb or extructed honey-comb, into the aqueous solution, acidifying the filter and washing and drying the filter.

The invention is described below in greater detail by way of example only with reference to the accompany drawings, in which

Fig. 1A is a perspective view showing a further embodiment of the composite deodorizing filter of the present invention;

Fig. 1B is a sectional view taken on line c-c' of Fig. 1A;

Fig. 2A is a perspective view showing a further embodiment of the composite deodorizing filter of the present invention;

Fig. 2B is a sectional view taken on line c-c' of Fig. 2A;

Fig. 3A is a perspective view showing a further embodiment of the composite deodorizing filter of the present invention;

Fig. 3B is a sectional view taken on line c-c' of Fig. 3A;

Fig. 4A is a perspective view showing a further embodiment of the composite deodorizing filter of the present invention;

Fig. 4B is a sectional view taken on line c-c' of Fig. 4A;

Fig. 5A is a perspective view showing a further embodiment of the composite deodorizing filter of the present invention;

Fig. 5B is a sectional view taken on line c-c' of Fig. 5A;

Fig. 6 is a graph for explaining a result of the adsorption qualification in the embodiment shown in Figs. 1A and 1B;

Fig. 7 is a graph for explaining a result of the adsorption qualification in the embodiment shown in Figs. 1A and 1B;

Fig. 8A is a perspective view illustrating a further embodiment of the composite deodorizing filter of the present invention;

Fig. 8B is a perspective view illustrating a further embodiment of the composite deodorizing filter of the present invention;

Fig. 9A is a perspective view illustrating an air conditioner as an apparatus to which the composite deodorizing filter of the present invention is applied;

Fig. 9B is a schematic structural view illustrating an air conditioner as an apparatus to which the composite deodorizing filter of the present invention is applied;

Fig. 10A is a perspective view showing an embodiment in which the composite deodorizing filter is installed in an air purifier;

Fig. 10B is a schematic structural view showing an embodiment in which the composite deodorizing filter is installed in an air purifier;

Fig. 11 is a perspective view showing an embodiment in which the composite deodorizing filter is installed in a vacuum cleaner;

Fig. 12A is a perspective view showing an embodiment in which the composite deodorizing filter is installed in a microwave range;

Fig. 12B is a schematic structural view showing an embodiment in which the composite deodorizing filter is installed in a microwave range;

Fig. 13 is a schematic structural view illustrating an embodiment of a composite deodorizing filter apparatus of the present invention, which is used in an air conditioner;

Fig. 14 is a schematic structural view illustrating another embodiment of a composite deodorizing filter apparatus of the present invention, which is used in an air conditioner;

Fig. 15 is a view showing the structure of metal (iron) phthalocyanine;

Fig. 16A is a view showing the structure of a dimer of metal phthalocyanine; and

Fig. 16B is a view showing the structure of a dimer of metal phthalocyanine.

Using the composite deodorizing filter of the invention, odor molecules are firstly absorbed by odor molecule adsorbing material mainly consisting of activated carbon. Therefore, deodorization is effectively carried out even in an air flowing environment. Leakage of odor due to separation from adsorbent can be prevented by decomposing the odor molecules separated from the activated carbon with oxidation catalytic reaction of the transition metal chelate compound adjacent to such adsorbent. The separated odor molecules are removed by decomposition reaction so that chemical equilibrium between the adsorption and separation of odor molecules in the activated carbon changes and separation and composition proceeds. Thus, the adsorbing ability of the activated carbon will not be saturated. It is thus possible to provide a deodorizing filter in which adsorption and decomposition of the odor molecules proceeds by two materials so that the deodorizing speed is fast and deodorizing effect is effective even in an air flowing environment, which is applicable to air conditioner and the like.

Provision of heating means which is bonded to the odor molecule adsorbent to prevent the lowering of the adsorbing speed due to the thermal dependence of separation and decomposition reaction speed of the odor molecules promotes the separation reaction of the odor molecules. The separated odor molecules are diffused into a filter made of the transition metal chelate compound so that they are rapidly decomposed. Therefore, the adsorbing speed reaches an equilibrium so that it will not become slow. Since the separation reaction is actively promoted, recovery of the odor molecule adsorbent is not necessary. Promotion of separation by heating may be conducted simultaneously with the deodorizing operation or intermittently. Alternatively, it may be conducted after completion of a deodorizing operation. It is thus possible to provide a deodorizing filter in which adsorption and decomposition of the odor molecules is promoted by both filter means and heating means so that the deodorizing speed is fast and deodorizing effect is effective even in an air flowing environment, which requires no recovery operation and can be used for an extended period of time and is applicable to air conditioners.

In order to prevent the fact from occurring that the humidity indoors is lowered and that the water content is removed from a deodorizing filter to dry the filter so that the deodorizing effect of the transition metal chelate compound is lowered, the deodorizing filter is constantly provided with a proper amount of water or humidity to enhance the deodorizing effect. A mechanism to inject water (preferably, deodorizing liquid) to the deodorizing filter is provided to keep the water content in the filter constant. A mechanism is provided to supply the filter with water by thermoelectric cooling the filter to dew-condensate water in air thereon.

The above-mentioned embodiments of the present invention will now be described in detail with reference to the drawings. Like references denote parts having like functions through drawings for describing the embodiment. Repeated description will be omitted.

In the embodiments of the invention, there is provided a composite deodorizing filter in which an odor molecule adsorbing material made of activated carbon or zeolite is provided with heating means, on which a transition metal chelate compound which exhibits a catalytic reaction for odor molecules is adjacent.

The composite deodorizing filter including heating means may be in the form as follows:

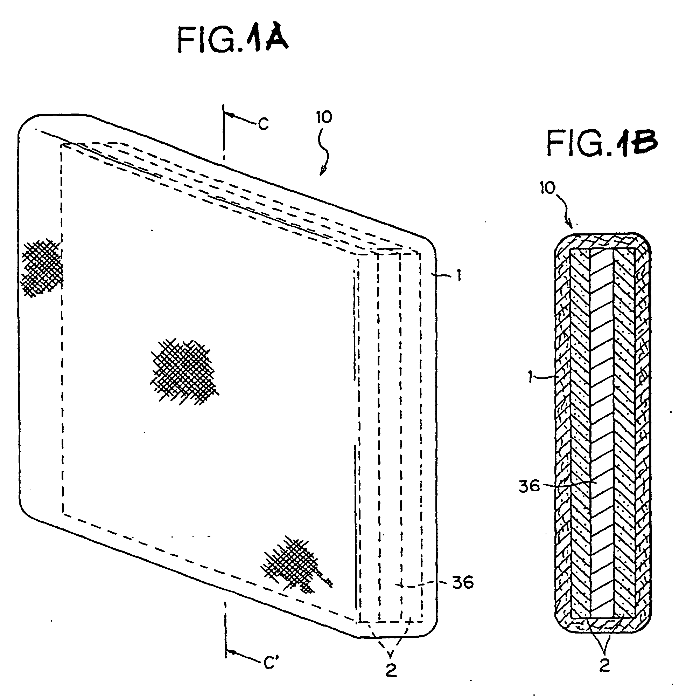

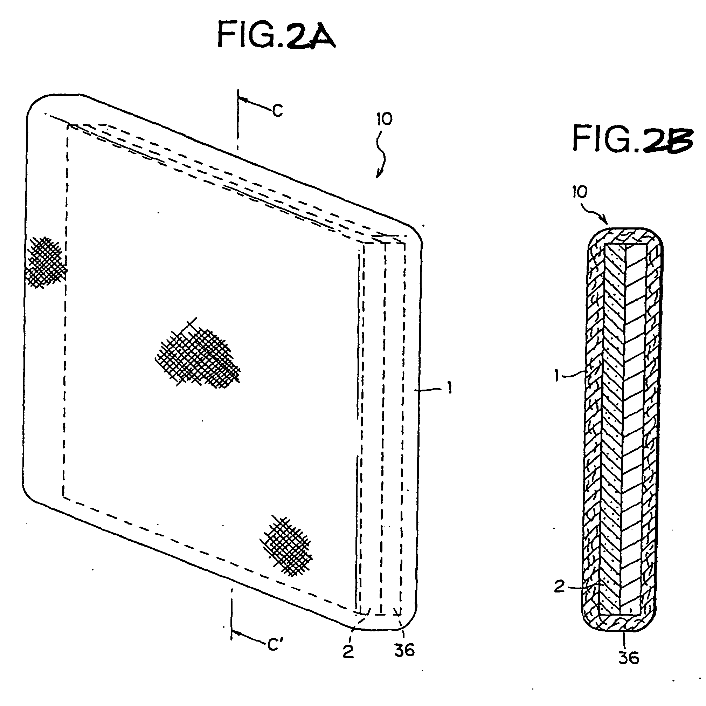

(1) Filter-like heating means and filter-like odor molecule adsorbing material are wrapped with a bag-like filter made of a transition metal chelate compound (Figs. 1A, 1B, 2A, 2B).

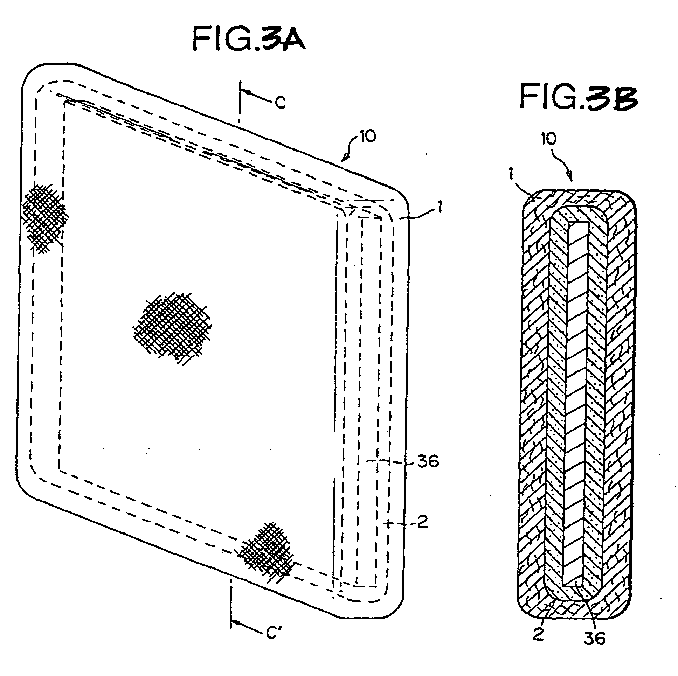

(2) Filter-like heating means is wrapped with a bag-like odor molecule adsorbing material filter, which is then wrapped with a bag-like transition metal chelate compound filter (Figs. 3A and 3B).

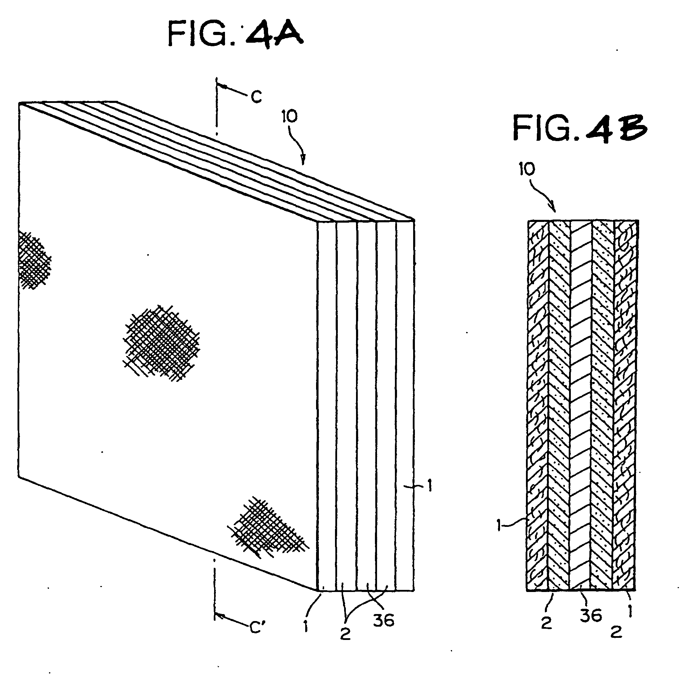

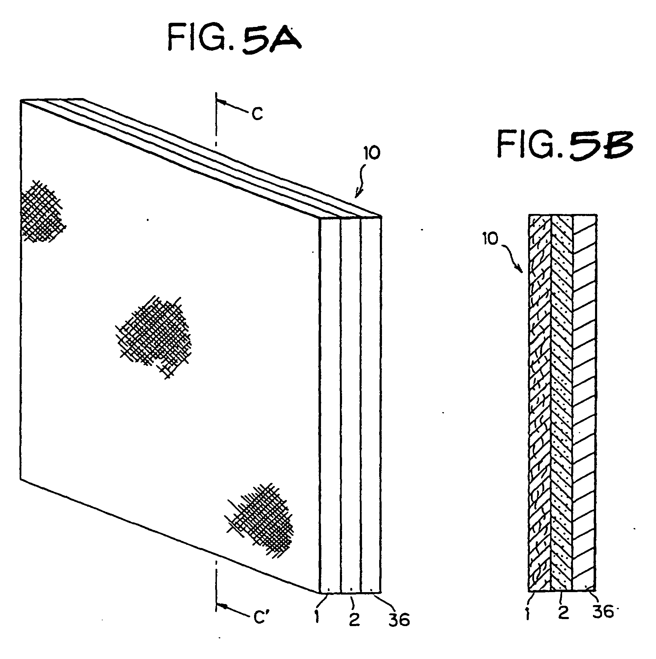

(3) Filter-like heating means, an odor molecule adsorbing material filter and a transition metal chelate compound filter are bonded to each other (Figs. 4A, 4B, 5A and 5B).

(4) A structure in which odor molecule adsorbing material fibers and transition metal chelate compound fibers are braided into filter-like heating means The filter-like heating means is used as an air-permeable substrate).

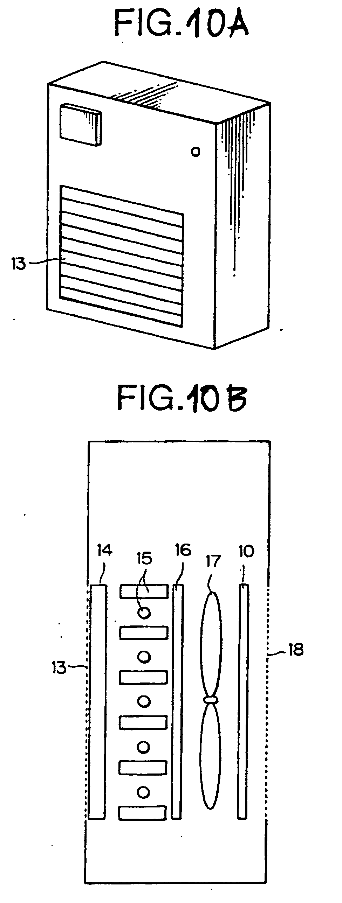

(5) A mixture of an odor molecule adsorbing material with the transition metal chelate compound or an odor molecule adsorbing material on which a transition metal chelate compound is carried is housed in a heat resistive air-permeable casing, which is in turn mounted on a filter-like heating means having an air permeability (the structure is similar to that shown in Figs. 8A and 8B).

(6) A structure in which powder or granules of odor molecule adsorbed decomposed material (a mixture of an odor molecule adsorbing material with the transition metal chelate compound or an odor molecule adsorbing material which is coated with a transition metal chelate compound (not shown).

A method of manufacturing the composite deodorizing filter of the embodiment as shown in Figs. 1A and 1B will be described.

One % of octacarboxyphthalocyanine iron solution was prepared by dissolving octacarboxyphthalocyanine iron in potassium hydroxide which was equivalent to the carboxyl groups of octacarboxyphthalocyanine iron. After a bag-like air-permeable unwoven fabric was dipped in the solution, it was lifted and then dipped in diluted hydrochloric acid solution so that it was neutralized. Then octacarboxyphthalocyanine iron was carried on the fabric by drying it with air. By inserting an activated carbon filter 2 of the odor molecule adsorbing material and a mesh-like heating plate 36 into an air permeable bag 1 on which the octacarboxyphthalocyanine iron was carried as shown in Figs. 1A and 1B. a composite deodorizing filter 10 having a structure that the heating means and the odor molecule adsorbing material was wrapped with a bag of the transition metal chelate compound was made.

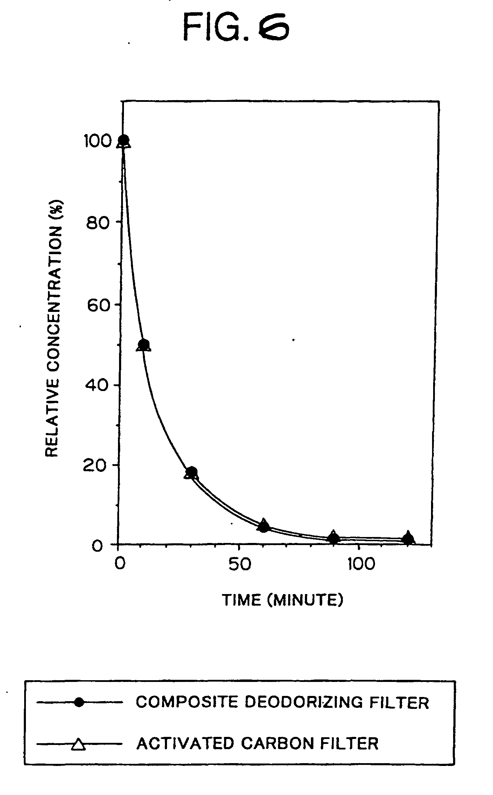

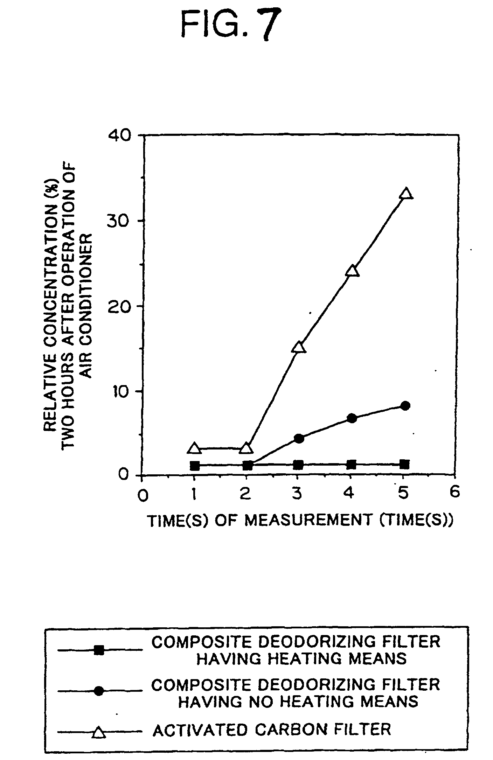

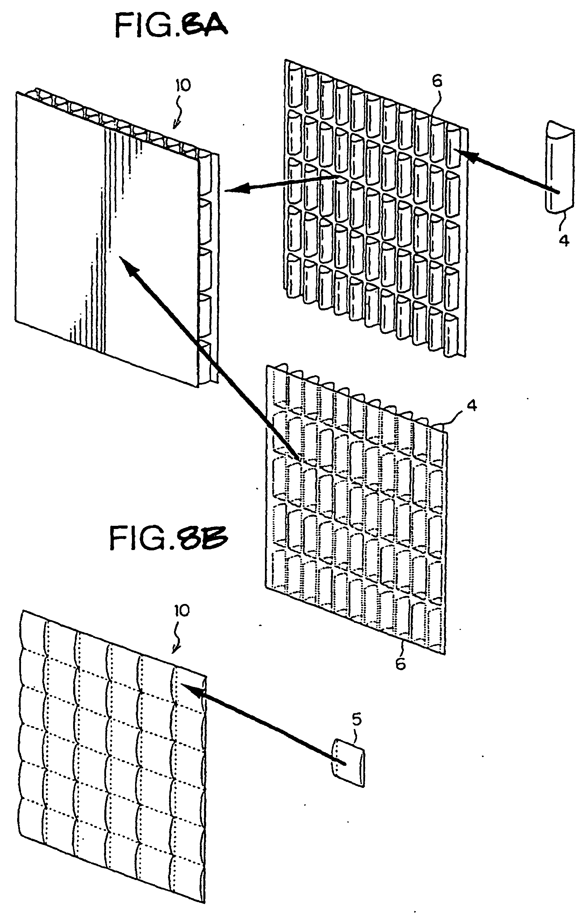

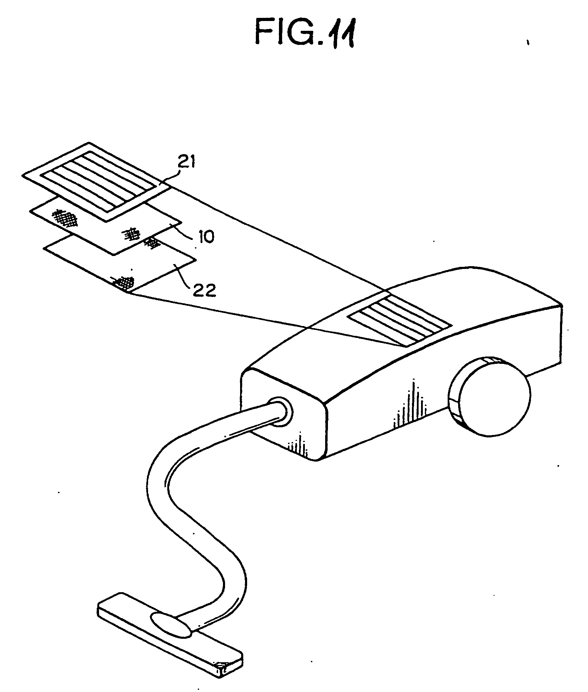

The manufactured composite deodorizing filter 10 was sandwiched between a dust collecting sandwiched filter 9 of an indoor unit of domestic use air conditioner and a heat exchanger 11 as shown in Figs. 9A and 9B. This filter mounted air conditioner was mounted in a qualification box (1 cubic meter). The air in the qualification box was adjusted to an atmosphere in the general home having a temperature of 25 °C and humidity of 50 % and hydrogen sulfide gas was admitted thereto. The hydrogen sulfide was sampled before and during the operation of the air-conditioner so that its concentration was measured with a detecting tube and a gas chromatography having a flame photometry detector. Measurement was conducted at an initial concentration of 80 ppm and 100 ppb when the detecting tube or the gaschromatography was used, respectively. Similar measurement was conducted for the activated carbon filter which was not wrapped with a filter-like octacarboxyphthalocyanine iron bag for comparison. Measurement showed that both filter exhibited deodorizing capability and decreases residual odor with the lapse of time as shown in Fig. 6.

Measurement was conducted for the other odors than hydrogen sulfide. Similar results were obtained. The odor remaining ratios (relative concentration) of respective odors after 120 minutes of filtering are shown in Table 1.

[Table 1]

|  | REMAINING ODOR RATIO (%) | | | |
|---|---|---|---|---|
|  | HYDROGEN SULFIDE | AMMONIA | TRIMETHYLAMINE | METHYL MERCAPTAN |
| COMPOSITE DEODORIZING FILTER | 1 | 4 | 3 | 5 |
| ACTIVATED CARBON FILTER | 3 | 10 | 5 | 3 |

A composite deodorizing filter which is shown in Figs. 1A and 1B was used. An activated carbon filter which did not have the octacarboxyphthalocyanine iron bag of the composite deodorizing filter shown in Figs. 1A and 1B was used.

Measurement was conducted with the detecting tube. The initial concentration of odors such as hydrogen sulfide, ammonia, trimethylamine and methyl mercaptan was 80 ppm, 400 ppm, 100 ppm and 300 ppm, respectively.

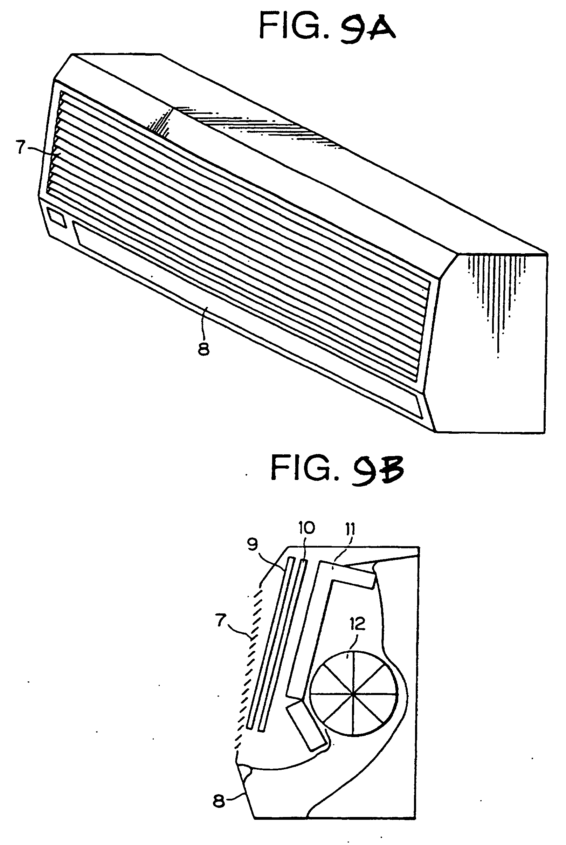

After measurement of the odor remaining ratio for 120 minutes, the operation of the air-conditioner was stopped and the heating plate 36 of the composite deodorizing filter was energized for 300 minutes. Then, measurement of the hydrogen sulfide concentration in the qualification box was conducted by admitting hydrogen sulfide gas again and by operating the air-conditioner without replacing any filter. Such a process comprising operating the air conditioner for two hours, thereafter heating the filter for 30 minutes and admitting hydrogen sulfide again was repeated for measuring the change in deodorizing ability of the filter with lapse of time. The repeated measurement was conducted by using the composite deodorizing filter with heating means of the present embodiment, the composite deodorizing filter having no heating means, in which the odor molecule adsorbing material was wrapped with a bag of the transition metal chelate compound, and single activated carbon filter for comparison of them. A result showed that the composite deodorizing filter of the present invention which was provided with heating means sustained higher deodorizing ability than the filter having not heating means even if the air conditioner was repeatingly operated as shown in Fig. 7. In particular, the prior art filter including only activated carbon exhibited remarkable lowering of the deodorizing ability. This is due to the fact that hydrogen sulfide which has been adsorbed by the activated carbon was saturated so that the filter loses its deodorizing ability. On the other hand, in the composite deodorizing filter the hydrogen sulfide which has been adsorbed by the activated carbon was deodorized with oxidation reaction with octacarboxyphthalocyanine iron when it was separated. In particular, the composite deodorizing filter including heating means sustained the adsorbing ability of the activated carbon filter since the separation of the odor components was promoted.

For last 30 minutes of 120 minute similar measurement, that is, after 90 minutes since the air-conditioner was operated, heating was conducted. The concentration of hydrogen sulfide after completion of the measurement of 120 minutes was increased by 5 % from that when the heating was initiated. The deodorizing ability was kept constant even if hydrogen sulfide was repeatingly admitted.

In such a manner, heating may be intermittently conducted during or after completion of the conditioner.

Similar result was obtained with the filter structure shown in Figs. 2A, 2B, 3A, and 3B. Since the heating plate 36 is in direct contact with the air-permeable bag 1 on which octacarboxyphthalocyanine iron is carried in the structure of Figs. 2A and 2B, the amount of heat from the heating plate 36 is lowered. Accordingly, the deodorizing ability after several repetitions of the admission of odor is lower by 10 % than that of the embodiments of Figs. 1A, 1B, 3A and 3B. Lowering of the deodorizing ability can be prevented by adjusting the heating period of time and the heat amount in practical air conditioner's operation.

Now, the embodiments of Figs. 4A, 4B, 5A and 5B will be described.

As shown in Figs. 4A and 4B, a mesh-like heating plate 36 was sandwiched between two activated carbon filter 2 and was then sandwiched between two sheets of air-permeable unwoven fabric 1 on which octacarboxyphthalocyanine iron was carried. Thus, the composite deodorizing filter 10 having an air permeability was manufactured.

The manufactured composite deodorizing filter 10 was mounted on an air conditioner, which was then installed in an qualification box to measure its deodorizing effect similarly to the embodiment shown in Figs. 1A and 1B. The measurement result showed that there was no significant difference from the result of the embodiment of Figs. 1A and 1B. The composite deodorizing filter 10 did not only exhibited a deodorizing effect, but also the deodorizing effect was continued even by repeating its operation.

Similar result was obtained when the filter having a structure which is shown in Figs. 5A and 5B was used.

An embodiment in which the fiber-like formed adsorbent and transition metal chelate compound are braided into the heating means will now be described.

An air-permeable composite deodorizing filter 10 was manufactured in which air-permeable fibers on which octacarboxyphthalocyanine iron was carried and fiber-like activated carbon were braided into a mesh-like heating plate.

The manufactured composite deodorizing filter 10 was mounted on an air conditioner, which was then installed in an qualification box to measure its deodorizing effect similarly to the embodiment shown in Figs. 1A and 1B. The measurement result showed that there is no significant difference from the result of the embodiment of Figs. 1A and 1B. The composite deodorizing filter did not only exhibited a deodorizing effect, but also the deodorizing effect was continued even by repeating its operation.

An embodiment in which a mesh-like heating plate is made of air-permeable porous paper to which cases in the embodiment shown in Figs. 8A and 8B are applied will be described.

One % of octacarboxyphthalocyanine iron solution was prepared by dissolving octacarboxyphthalocyanine iron in potassium hydroxide which was equivalent to the carboxyl groups of octacarboxyphthalocyanine iron. After granular activated carbon was dipped in the solution, it was lifted and neutralized by dipping diluted hydrochloric acid solution. It was dried with air so that octacarboxyphthalocyanine iron was carried. The activated carbon granules were placed into cases 4 made of air-permeable porous paper as shown in Figs. 8A and 8B. A composite deodorizing filter 10 was manufactured by applying the cases 4 to a mesh-like heating plate (which is described as an air-permeable porous paper 6 in the above-mentioned embodiment using no heating means). Two composite deodorizing filters may be used in such manner that the same sides face to each other. Alternatively, a heating plate may be inserted into a bag of air-permeable porous paper as shown in Fig. 8B and activated carbon granules on which octacarboxyphthalocyanine iron was carried was charged into the bag. The granules may be evenly distributed by perforating the bag. Thus, the composite deodorizing filter 10 may be manufactured.

The manufactured composite deodorizing filter 10 was sandwiched between a dust precipitating filter 9 of an indoor unit of domestic use air conditioner and a heat exchanger 11 as shown in Figs. 9A and 9B. This filter mounted air conditioner was mounted in a qualification box (1 cubic meter). The air in the qualification box was adjusted to an atmosphere in the general home having a temperature of 25 °c and humidity of 60 % and hydrogen sulfide gas was admitted thereto. The hydrogen sulfide was sampled before and during the operation of the air-conditioner so that its concentration was measured with a detecting tube and a gas chromatography having a flame photometry detector. Similar measurement was conducted for a single activated carbon filter including no octacarboxyphthalocyanine iron.

Both filters deodorized hydrogen sulfide. It took 120 minutes for the relative concentration to reach 3 % due to pressure loss. However, the composite deodorizing filter of the present embodiment did not lose its deodorizing ability similarly to the embodiment of Figs. 1A and 1B even if its operation was repeated.

As an example of the apparatus using the composite deodorizing filter having heating means, an air-purifier was formed in which the composite deodorizing filter 10 of the embodiment shown in Figs. 1A and 1B was disposed between the fan 15 and air blowing outlet 18 as shown in Figs. 10A and 10B. The composite deodorizing filter 10 may be disposed between the dust precipitating filter 16 and the fan 17. The deodorizing effect of the air-purifier in which the composite deodorizing filter 10 was disposed was sustained even if it was used for three months.

As an other example of the apparatus, a dehumidifier was formed in which the composite deodorizing filter 10 of the embodiment shown in Figs. 1A and 1B was disposed in the rear of the exhaust inlet and the dust precipitating filter. The deodorizing effect was sustained even if it was used for three months.

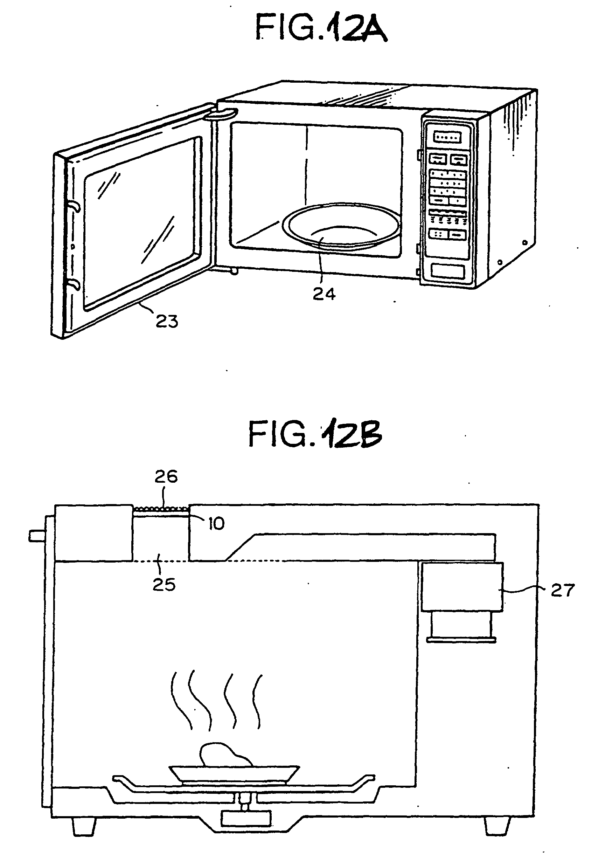

As an other example of the apparatus, a vacuum cleaner was formed in which the composite deodorizing filter 10 of the embodiment shown in Figs. 1A and 1B are disposed between the exhaust outlet 21 and the dust precipitating filter 22 as shown in Fig. 11. The deodorizing effect was sustained even if it was used for one month.

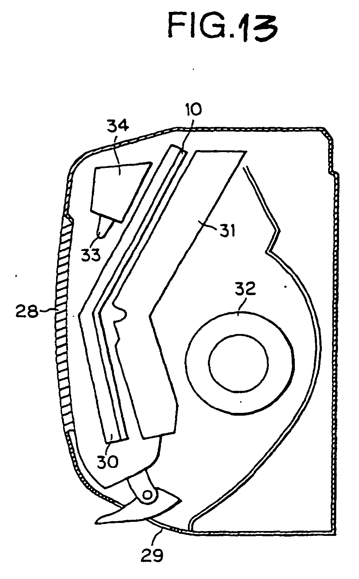

Another example of the apparatus, a microwave range was formed in which the composite deodorizing filter 10 of the embodiment of Figs. 1A and 1B was disposed in front of the exhaust outlet 26 as shown in Figs. 12A and 12B. The odor which was generated in the range was deodorized when it was discharged via the exhaust outlet 25 via the exhaust guide 26. The deodorizing effect was sustained even if the filter was used for one month. If only the dried foods were heated by the range, the deodorizing effect was lowered since the space in the microwave range was in the very dried condition.

An embodiment of the composite deodorizing filter apparatus including means for providing the apparatus with a proper amount of water or humidity will be described.

Fig. 13 is a schematic structural view explaining an embodiment of the composite deodorizing filter of the present invention and showing an air-conditioner using the composite filter apparatus. In the drawings, reference numeral 28 denotes an air intake inlet; 29 an air blowing outlet; 30 a dust precipitating filter; 31 a heat exchanger; 32 a fan; 33 a water injecting nozzle; and 34 a water reserving container. The composite deodorizing filter apparatus of the present embodiment has as components a composite deodorizing filter, water reserving container 34, and water injecting nozzle 33 and includes a mechanism for supplying the composite deodorizing filter 10 with water.

The composite deodorizing filter was manufactured by a process which was similar to the foregoing method. The water injecting nozzle 33 for injecting water upon the surface of the composite deodorizing filter 10 was mounted upon the water reserving container 34 and was adapted to inject water when the water content in the composite filter 10 is lowered. The produced composite deodorizing filter apparatus was mounted on the dust precipitating filter 30 of the indoor unit of domestic use air-conditioner as shown in Fig. 13.

An air conditioner on which the composite deodorizing filter apparatus was mounted was installed on a deodorization qualification box (1 cubic meter) for measuring the deodorizing effect. The temperature and humidity in the qualification box was adjusted to 25 °C and 20 % or 50 %, respectively. Hydrogen sulfide gas was admitted to the box and sampling was conducted before and after the operation of the air-conditioner. The hydrogen sulfide gas concentration was measured by means of a detecting tube. The initial concentration of the hydrogen sulfide gas was made 60 ppm.

A result of the measurement showed that hydrogen sulfide was completely deodorized after 30 minutes by means of the composite deodorizing filter when the humidity was 50 %. No hydrogen sulfide was detected by the detecting tube when the humidity was 20 %. However, the hydrogen sulfide concentration was 5 ppm after 30 minutes when the humidity was 20 %. The hydrogen sulfide gas could be completely deodorized after 90 minutes. The water content in the composite deodorizing filter was increased by injecting water into the composite deodorizing filter 10 before the admission of the hydrogen sulfide gas when the humidity was 20 %. As a result the hydrogen sulfide could be completely deodorized after 30 minutes similarly to the data when the humidity was 50 %.

Another embodiment of the composite deodorizing filter apparatus will now be described.

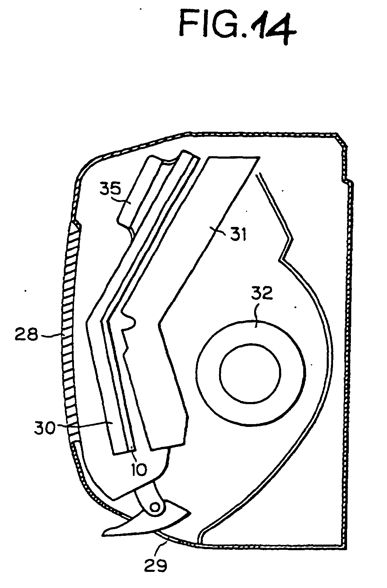

Fig. 14 is a schematic structural view explaining another embodiment of the composite deodorizing filter apparatus of the present invention and showing an air conditioner using the composite deodorizing filter apparatus. In the drawing of Fig. 14, reference numeral 35 denotes a Peltier effect element.

Now, a process for producing a deodorizing filter having a metallic honeycomb structure will be described.

A potassium hydroxide aqueous solution containing octacarboxyphthalocyanine iron at 1 % was prepared. After a metal plate (which was preliminarily coated on the surface thereof with zeolite powders with a binder) of a honeycomb structure having a low pressure loss was dipped in the aqueous solution, it was lifted and the dipped in the diluted hydrochloric aqueous solution so that it was neutralized. Then, it was sufficiently washed so that octacarboxyphthalocyanine iron was carried on the surface of the metallic filter.

A composite deodorizing filter apparatus was formed in which a Peltier effect element 35 for cooling the composite deodorizing filter 10 was mounted thereon. The temperature on the Peltier element 35 was lowered by the thermoelectric coding to cause the humidity in air to be dew-condensated so that the composite deodorizing filter 10 can be supplied with water.

The produced composite deodorizing filter apparatus was mounted on the dust precipitating filter 30 of the indoor unit of the domestic use air conditioner, which was installed on an air-conditioner deodorization qualification box (1 cubic meter) as shown in Fig. 14.

An air conditioner on which the composite deodorizing filter apparatus was mounted was installed on a deodorization qualification box (1 cubic meter) for measuring the deodorizing effect. The temperature and humidity in the qualification box was adjusted to 25 °C and 20 % or 50 %, respectively. Hydrogen sulfide gas was admitted to the box and sampling was conducted before and after the operation of the air-conditioner. The hydrogen sulfide gas concentration was measured by means of a detecting tube. The initial concentration of the hydrogen sulfide gas was made 70 ppm.

When the humidity was 50 %, a result of the measurement showed that hydrogen sulfide was completely deodorized after 30 minutes by means of the composite deodorizing filter, and no hydrogen sulfide was detected by the detecting tube. However, when the humidity was 20 %, the hydrogen sulfide concentration was 10 ppm after 30 minutes, and hydrogen sulfide completely deodorized after 100 minutes. The water content in the composite deodorizing filter was increased by injecting water into the composite deodorizing filter 10 before the admission of the hydrogen sulfide gas when the humidity was 20 %. As a result the hydrogen sulfide could be completely deodorized after 30 minutes by increasing the water content in the filter.

It is necessary that the metal phthalocyanine be bonded with a filter carrier in a monomer state since deodorization is achieved by adsorption of the odor molecules to the metal phthalocyanine complex in a deodorizing filter including the above-mentioned metal phthalocyanine complex which is mixed with deodorizing molecule adsorbent such as activated carbon or zeolite. Accordingly, the metal phthalocyanine is chemically bonded with inorganic materials or polymer compounds to prevent dimers from being formed.

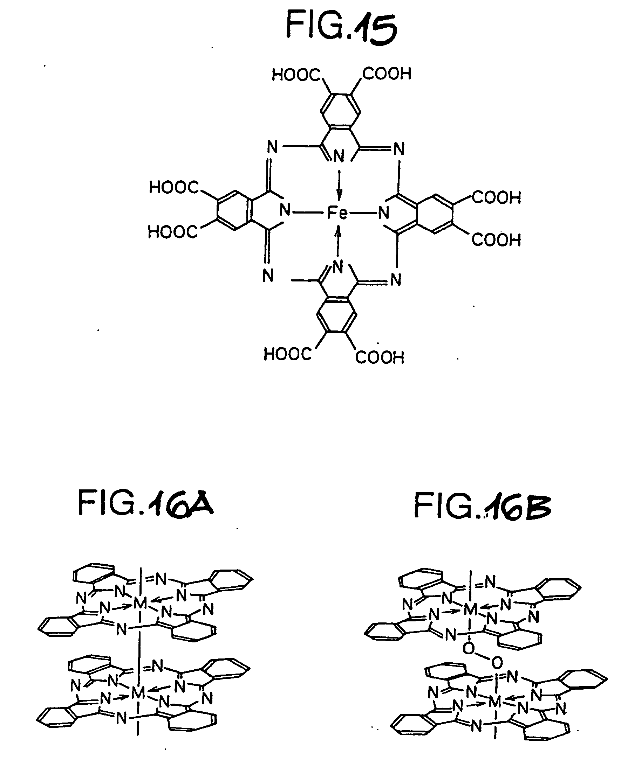

It has been reported that molecules having a number of carboxyl groups of the metal phthalocyanine derivatives is unliable to form dimmers due to its steric hindrance. The molecular structure of octacarbonate-iron phthalocyanine having 8 carboxyl groups is shown in Fig. 15. However, it is found that the metal phthalocyanine molecules will gradually form dimers in an alkaline aqueous solution with the lapse of time.

The structure of a dimer of the metal phthalocyanine complex is shown in Figs. 16A and 16B. Since the metal phthalocyanine per se is planar in configuration, it will ultimately form a metal phthalocyanine dimer as shown in Fig. 16A and metal phthalocyanine- $\mu$ -oxodimer. Therefore, a central metal M becomes unliable to accept electrons of the other materials so that the catalytic activity will be lowered.

Embodiments of a process for producing a deodorizing filter are as follows, in which no dimers of the metal phthalocyanine will be formed in an alkaline aqueous solution in which metal phthalocyanine is dissolved.

The inventors of the present application have discovered that it is oxygen dissolved in an alkaline aqueous solution of polycarboxylic-acid-metal-phthalocyanine that causes metal phthalocyanine to form dimers and have found that dimerization from monomer to dimer can practically be suppressed by removing oxygen dissolved in the aqueous solution and further have found that bubbling with inert gas or addition of sodium sulfite anhydride salt is effective to remove oxygen dissolved in the alkaline aqueous solution.

In order to solve the above mentioned problems, the process for producing a deodorizing filter or the present invention is characterized in that it comprises the steps of: adjusting an alkaline aqueous solution in which polycarboxylic-acid-metal-phthalocyanine is dissolved; removing oxygen dissolved in the alkaline aqueous solution; carrying polycarboxylic-acid-metal-phthalocyanine on a filter carrier by dipping the filter carrier in the alkaline aqueous solution; treating in an acid bath the filter carrier to which the polycarboxylic-acid-metal-phthalocyanine has been carried by the above-mentioned step; and thereafter washing the treated filter carrier with water and drying the same.

Preferably, the step for removing oxygen dissolved in the alkaline aqueous solution includes bubbling the alkaline aqueous solution with a gas or adding sodium sulfite anhydride to the alkaline aqueous solution. Preferably, inert gas such as nitrogen or argon is used as a bubbling gas.

Said filter carrier is made of unwoven fabric, paper honeycomb, ceramic fiber honeycomb or extruded honeycomb.

Now, a process for producing a deodorizing filter of the present invention will be described by way of embodiments in detail.

A process for producing a deodorizing filter using unwoven fabric having an air permeability in accordance with a first embodiment will be described. Dissolved oxygen was removed by bubbling with nitrogen gas an alkaline aqueous solution in which potassium hydro oxide is dissolved.

An aqueous solution was prepared by dissolving octacarboxyphthalocyanine iron in the alkaline aqueous solution at a concentration of 1 % so that phthalocyanine iron serves as alkaline salt. After dipping a sheet of unwoven fabric having an air permeability in the alkaline aqueous solution, the unwoven fabric was lifted and then dipped into diluted hydrochloric acid aqueous solution for neutralization. Subsequently, the fabric was sufficiently washed with water so that the octacarboxyphthalocyanine iron was carried on the unwoven fabric.

The produced deodorizing filter was mounted on an indoor dust precipitating filter unit of a domestic use air-conditioner. The air conditioner on which the deodorizing filter was mounted was installed on a deodorizing qualifying box (one cubic meter) so that its deodorizing effect was determined. The temperature in the qualifying box was at 25 °C and the humidity was adjusted at 30 %. Hydrogen sulfide gas was introduced into the box. Sampling was conducted before and after the operation of the air-conditioner to measure concentration of hydrogen sulfide with a gas detecting tube (manufactured by GAS TECK Co., Ltd.).

The initial concentration of hydrogen sulfide was at 15 ppm. After 30 minutes, hydrogen sulfide was completely eliminated with the deodorizing filter. No hydrogen sulfide was detected.

[Embodiment of a process for producing a deodorizing filter 2]

A process for producing a deodorizing filter using a honeycomb structure made of ceramic fiber paper in accordance with a second embodiment will be described. A honeycomb structure made of ceramic fiber paper is excellent in heat resistance and corrosion resistance and is lighter than ceramic honeycomb structure which was produced by extrusion and has a low pressure loss. HANIKURU LT (manufactured by Nichiasu Co., Ltd.) which is a honeycomb structure made of ceramic fiber paper was used.

Sodium sulfite anhydride was dissolved to pure water at 5 % concentration. After removing dissolved oxygen, an alkaline aqueous solution was prepared by dissolving octacarboxyphthalocyanine iron in an alkaline aqueous solution at a concentration of 1 %, the pH of which was adjusted by addition of potassium oxide. After the honeycomb structure was dipped in the prepared alkaline aqueous solution, it was lifted up for drying. Then, the honeycomb structure was dipped into diluted hydrochloric acid aqueous solution. Thereafter, it was washed with water. A deodorizing filter was thus manufactured.

The produced deodorizing filter was mounted on an indoor dust precipitating filter unit of a domestic use air-conditioner. The air-conditioner on which the deodorizing filter was mounted was installed on a deodorizing qualifying box (one cubic meter) so that its deodorizing effect was determined. The temperature in the qualifying box was at 25 °C and the humidity was adjusted at 30 %. Hydrogen sulfide gas was introduced into the box. Sampling was conducted before and after the operation of the air conditioner to measure concentration of hydrogen sulfide with a gas detecting tube (manufactured by GAS TECK Co., Ltd.).

The initial concentration of hydrogen sulfide was at 15 ppm. After 30 minutes, hydrogen sulfide was completely eliminated with the deodorizing filter. No hydrogen sulfide was detected.

[Embodiment of a process for producing a deodorizing filter 3]

A process for producing a deodorizing filter having a metallic honeycomb structure in accordance with a third embodiment will be described. A honeycomb filter of aluminum having a surface on which hydrophobic zeolite was carried with an inorganic binder was used as the honeycomb structure. An alkaline aqueous solution in which octacarboxyphthalocyanine iron and potassium oxide, each having a concentration of 1 % was dissolved was prepared. After a metallic honeycomb structure having a low pressure loss was dipped in the alkaline aqueous solution, it was lifted up for drying. Then, it was dipped into diluted hydrochloric acid aqueous solution for neutralization. Then it was sufficiently washed with pure water so that octacarboxyphthalocyanine iron was carried on the filter surface to prepare a deodorizing filter.

The produced deodorizing filter was mounted on an indoor dust precipitating filter unit of a domestic use air-conditioner. The air-conditioner on which the deodorizing filter was mounted was installed on a deodorizing qualifying box (one cubic meter) so that its deodorizing effect was determined. The temperature in the qualifying box was at 25 °C and the humidity was adjusted at 30 %. Hydrogen sulfide gas was introduced into the box. Sampling was conducted before and after the operation of the air conditioner to measure concentration of hydrogen sulfide with a gas detecting tube (manufactured by GAS TECK Co., Ltd.).

The initial concentration of hydrogen sulfide was at 15 ppm. After 30 minutes, hydrogen sulfide was completely eliminated with the deodorizing filter. No hydrogen sulfide was detected.

Although bubbling with nitrogen gas or addition of sodium sulfite anhydride salt to remove dissolved oxygen has been disclosed in the foregoing embodiments, the present invention is not limited to these methods. It is of course possible to appropriately choose any other method if it can achieve similar object.

The advantages of the present invention are as follows.

(1) There is provided a filter in which its adsorption performance for odor is so excellent even in an environment in which air containing odor flows and a high deodorizing effect is provided and the adsorption performance which is stable with the lapse of time is maintained and necessity of time and labor consuming filter exchanging operation is obviated since its service life is long, which is capable of providing a comfortable environment.

(2) The adsorption performance of the adsorbent can be stably maintained by providing heating means.

(3) Lowering of the adsorption performance of the composite deodorizing filter in a very dried atmosphere is prevented by providing water supply means for supplying the composite deodorizing filter with water so that a stable adsorption performance can be maintained irrespective of the humidity conditions.

(4) Since metal phthalocyanine will not be dimerized by using a dust precipitating filter of an air-conditioner on which is mounted a deodorizing filter which was produced in accordance with the present invention for effectively conducting deodorization of indoor air, it is possible to provide a deodorizing filter having a deodorizing effect even in an indoor environment in which air flows without deteriorating its deodorizing effect.

## Claims

1. A composite deodorizing filter (10) having deodorizing means including an adsorbent (2) for odor molecules and a transition metal chelate for catalysing oxidation of odor molecules and **characterized by** including also air permeable heating means (36) integrated with and capable of heating the deodorizing means.

2. A composite deodorizing filter according to claim 1 which comprises powdery or granular adsorbent bonded to the heating means and wherein the transition metal chelate is carried on the heating means (36) and the adsorbent.

3. A composite deodorizing filter according to claim 1, wherein the deodorizing means comprises powdery or granular adsorbent and powdery or granular transition metal chelate, the deodorizing means being contained in a casing mounted on the heating means.

4. A composite deodorizing filter according to claim 1, wherein the deodorizing means comprises powdery or granular adsorbent which carries the transition metal chelate, the deodorizing means being contained in a casing mounted on the heating means.

5. A composite deodorizing filter according to claim 1, wherein the deodorizing means comprises an air permeable adsorbent-containing body (2) and an air permeable transition metal chelate-containing body (1), the heating means, the adsorbent-containing body (2) and the transition metal chelate-containing body (1) being arranged in sequence.

6. A composite deodorizing filter according to claim 1, wherein the deodorizing means comprises an air permeable adsorbent-containing body (2) and an air permeable transition metal chelate-containing body (1), a first transition metal chelate-containing body, a first adsorbent-containing body, the heating means, a second adsorbent-containing body and a second transition metal chelate-containing body being arranged in sequence.

7. A composite deodorizing filter according to claim 1, wherein an air permeable adsorbent-containing body (2) is bonded to the heating means and an air permeable transition metal chelate-containing body (1) is wrapped around the adsorbent-containing body and the heating means.

8. A composite deodorizing filter according to claim 1, wherein the heating means is sandwiched between two air permeable adsorbent-containing bodies (2) and an air permeable transition metal chelate-containing body (1) is wrapped around the sandwich.

9. A composite deodorizing filter according to claim 1, wherein an air permeable adsorbent-containing body (2) is wrapped around the heating means and an air permeable transition metal chelate-containing body (1) is wrapped around the adsorbent-containing body.

## Patentansprüche

1. Verbund-Desodorisierungsfilter (10) mit einem Desodorisierungsmittel, welches ein Adsorptionsmittel (2) für Geruchsmoleküle und ein Übergangsmetallchelat zum Katalysieren der Oxidation von Geruchsmolekülen enthält, und **dadurch gekennzeichnet ist, dass** er auch ein luftdurchlässiges Heizmittel (36) enthält, das integriert mit den Desodorisierungsmitteln ausgebildet ist und geeignet ist, diese zu heizen.

2. Verbund-Desodorisierungsfilter nach Anspruch 1, welcher ein an das Heizmittel gebundenes, pulvriges oder granuläres Adsorptionsmittel umfasst, und bei welchem das Übergangsmetallchelat auf dem Heizmittel (36) und dem Adsorptiosmittel getragen ist.

3. Verbund-Desodorisierungsfilter nach Anspruch 1, bei welchem das Desodorisierungsmittel ein pulvriges oder granuläres Adsorptionsmittel und ein pulvriges oder granuläres Übergangsmetallchelat umfasst, wobei das Desodorisierungsmittel in einem auf dem Heizmittel montierten Gehäuse enthalten ist.

4. Verbund-Desodorisierungsfilter nach Anspruch 1, bei welchem das Desodorisierungsmittel ein pulvriges oder granuläres Adsorptionsmittel umfasst, welches das Übergangsmetallchelat trägt, wobei das Desodorisierungsmittel in einem auf dem Heizmittel montierten Gehäuse enthalten ist.

5. Verbund-Desodorisierungsfilter nach Anspruch 1, bei welchem das Desodorisierungsmittel einen luftdurchlässigen, Adsorptionsmittel-enthaltenden Körper (2) und einen luftdurchlässigen. Übergangsmetallchelat-enthaltenden Körper (1) umfasst, wobei das Heizmittel, der Adsortionsmittel-enthaltende Körper (2) und der Übergangsmetallchelat-enthaltende Körper (1) in Folge angeordnet sind.

6. Verbund-Desodorisierungsfilter nach Anspruch 1, bei welchem das Desodorisierungsmittel einen luftdurchlässigen, Adsorptionsmittel-enthaltenden Körper (2) und einen luftdurchlässigen. Übergangsmetallchelat-enthaltenden Körper (1) umfasst, wobei ein erster Übergangsmetallchelat-enthaltender Körper (1), ein erster Adsorptionsmittel-enthaltender Körper, das Heizmittel, ein zweiter Adsorptionsmittel-enthaltender Körper und ein zweiter Übergangsmetallchelat-enthaltender Körper in Folge angeordnet sind.

7. Verbund-Desodorisierungsfilter nach Anspruch 1, bei welchem ein luftdurchlässiger, Adsorptionsmittel-enthaltender Körper (2) an das Heizmittel gebunden ist, und ein luftdurchlässiger, Übergangsmetallchelat-enthaltender Körper (1) um den Adsorptionsmittel-enthaltenden Körper und das Heizmittel gewickelt ist.

8. Verbund-Desodorisierungsfilter nach Anspruch 1, bei welchem das Heizmittel zwischen zwei luftdurchlässigen. Adsorptionsmittel-enthaltenden Körpern (2) in Sandwich-Bauweise angeordnet ist und ein luftdurchlässiger, Übergangsmetallchelat-enthaltender Körper (1) um das Sandwich gewickelt ist.

9. Verbund-Desodorisierungsfilter nach Anspruch 1, bei welchem ein luftdurchlässiger, Adsorptionsmittel-enthaltender Körper (2) um das Heizmittel gewickelt ist und ein luftdurchlässiger, Übergangsmetallchelat-enthaltender Körper (1) um den Adsorptionsmittel-enthaltenden Körper gewickelt ist.

**Revendications**

1. Filtre désodorisant composite (10) ayant des moyens désodorisants comprenant un adsorbant (2) pour des molécules odorantes et un chélate de métal de transition pour catalyser l'oxydation de molécules odorantes et **caractérisé en ce qu'**il comprend également des moyens de chauffage perméables à l'air (36) qui y sont intégrés aux moyens désodorisants et capables de chauffer ces derniers.

2. Filtre désodorisant composite selon la revendication 1, qui comprend un adsorbant pulvérulent ou granulaire lié aux moyens de chauffage et dans lequel le chélate de métal de transition est supporté par les moyens de chauffage (36) et l'adsorbant.

3. Filtre désodorisant composite selon la revendication 1, dans lequel les moyens désodorisants comprennent un adsorbant pulvérulent ou granulaire et un chélate de métal de transition pulvérulent ou granulaire, les moyens désodorisants étant contenus dans un boîtier monté sur les moyens de chauffage.

4. Filtre désodorisant composite selon la revendication 1, dans lequel les moyens désodorisants comprennent un adsorbant pulvérulent ou granulaire qui supporte le chélate de métal de transition, les moyens désodorisants étant contenus dans un boîtier monté sur les moyens de chauffage.

5. Filtre désodorisant composite selon la revendication 1, dans lequel les moyens désodorisants comprennent un corps (2) contenant un adsorbant perméable à l'air et un corps (1) contenant un chélate de métal de transition perméable à l'air, les moyens de chauffage, le corps (2) contenant un adsorbant et le corps (1) contenant un chélate de métal de transition étant placés les uns à la suite des autres.

6. Filtre désodorisant composite selon la revendication 1, dans lequel les moyens désodorisants comprennent un corps (2) contenant un adsorbant perméable à l'air et un corps (1) contenant un chélate de métal de transition perméable à l'air, un premier corps contenant un chélate de métal de transition, un premier corps contenant un adsorbant, les moyens de chauffage, un second corps contenant un adsorbant et un second corps contenant un chélate de métal de transition étant placés les uns à la suite des autres.

7. Filtre désodorisant composite selon la revendication 1, dans lequel un corps (2) contenant un adsorbant perméable à l'air est lié aux moyens de chauffage et un corps (1) contenant un chélate de métal de transition perméable à l'air est enroulé autour du corps contenant un adsorbant et les moyens de chauffage.

8. Filtre désodorisant composite selon la revendication 1, dans lequel les moyens de chauffage sont placés en sandwich entre deux corps (2) contenant un adsorbant perméable à l'air et un corps (1) contenant un chélate de métal de transition perméable à l'air est enroulé autour du sandwich.

9. Filtre désodorisant composite selon la revendication 1, dans lequel un corps (2) contenant un adsorbant perméable à l'air est enroulé autour des moyens de chauffage et un corps (1) contenant un chélate de métal de transition perméable à l'air est enroulé autour du corps contenant un adsorbant.

# FIG.1A

# FIG.1B

# FIG.2A

# FIG.2B

# FIG.3A

# FIG.3B

FIG. 4A

C

10

1

1 2 36

C'

FIG. 4B

10

1

2 36 1 2

# FIG. 5A

# FIG. 5B

# FIG. 6

Graph — X-axis: TIME (MINUTE), Y-axis: RELATIVE CONCENTRATION (%)

Legend:
- COMPOSITE DEODORIZING FILTER (●)
- ACTIVATED CARBON FILTER (△)

# FIG. 7

**RELATIVE CONCENTRATION (%) TWO HOURS AFTER OPERATION OF AIR CONDITIONER** (y-axis: 0, 10, 20, 30, 40)

**TIME(S) OF MEASUREMENT (TIME(S))** (x-axis: 0, 1, 2, 3, 4, 5, 6)

Legend:
- ■ COMPOSITE DEODORIZING FILTER HAVING HEATING MEANS
- ● COMPOSITE DEODORIZING FILTER HAVING NO HEATING MEANS
- △ ACTIVATED CARBON FILTER

## FIG.8A

## FIG.8B

# FIG. 9A

# FIG. 9B

# FIG.10A

# FIG.10B

# FIG.11

# FIG.12A

# FIG.12B

# FIG.13

# FIG.14

# FIG.15

# FIG.16A

# FIG.16B